# EUROPEAN PATENT SPECIFICATION

(11) **EP 4 316 525 B1**
(45) Date of publication and mention of the grant of the patent: **09.12.2026**
(21) Application number: 22791099.9
(22) Date of filing: 21.04.2022
(51) Int. Cl.: A61K 47/54, A61K 31/7088, A61K 47/60, A61P 35/00

(54) **CONJUGATE, PREPARATION METHOD THEREFOR AND USE THEREOF**
KONJUGAT, HERSTELLUNGSVERFAHREN DAFÜR UND VERWENDUNG DAVON
CONJUGUÉ, SON PROCÉDÉ DE PRÉPARATION ET SON UTILISATION

(30) Priority: 23.04.2021 CN 202110442394
(43) Date of publication of application: 07.02.2024
(73) Proprietor: Cosychem Biotechnology (TIANJIN) Co., Ltd., Tianjin 300457, (CN)
(72) Inventor: XI, Zhen, Tianjin 300071 (CN); YANG, Chao, Tianjin 300071 (CN)
(74) Representative: reuteler & cie SA
(86) International application number: PCT/CN2022/088173
(87) International publication number: WO 2022/222993

(56) References cited:
- WO-A1-2012/030745
- WO-A1-2018/076025
- WO-A2-2011/100131
- WO-A2-2012/024526
- WO-A2-2020/146521
- CN-A- 113 304 274
- US-A1- 2009 124 571
- US-A1- 2012 130 045
- WANYI TAI ET AL: "Synthetic Polymer Tag for Intracellular Delivery of siRNA", ADVANCED BIOSYSTEMS, vol. 2, no. 7, 29 May 2018 (2018-05-29), pages n/a, XP072281396, ISSN: 2366-7478, DOI: 10.1002/ADBI.201800075
- MCNAMARA JAMES O ET AL: "Cell type-specific delivery of siRNAs with aptamer-siRNA chimeras", NATURE BIOTECHNOLOGY, vol. 24, no. 8, 25 June 2006 (2006-06-25), pages 1005 - 1015, XP002397468, ISSN: 1087-0156, DOI: 10.1038/NBT1223
- "Proceedings of the World Molecular Imaging Congress 2013, Savannah, Georgia, September 18-21, 2013", MOLECULAR IMAGING & BIOLOGY, vol. 15, no. Suppl 1, 23 November 2013 (2013-11-23), pages 2 - 1590, XP037137046, ISSN: 1536-1632, [retrieved on 20131123], DOI: 10.1007/S11307-013-0703-2
- ZHIHANG CHEN ET AL: "PSMA-Targeted Theranostic Nanoplex for Prostate Cancer Therapy", ACS NANO, vol. 6, no. 9, 25 September 2012 (2012-09-25), pages 7752 - 7762, XP055135441, ISSN: 1936-0851, DOI: 10.1021/nn301725w
- CHERNIKOV IVAN V ET AL: "Fluorophore Labeling Affects the Cellular Accumulation and Gene Silencing Activity of Cholesterol-Modified siRNAs In Vitro.", vol. 29, no. 1, 28 December 2018 (2018-12-28), pages 33 - 43, XP093302368, ISSN: 2159-3345, Retrieved from the Internet <URL:https://www.liebertpub.com/doi/pdf/10.1089/nat.2018.0745> DOI: 10.1089/nat.2018.0745
- REKA A. HARASZTI ET AL: "5?-Vinylphosphonate improves tissue accumulation and efficacy of conjugated siRNAs in vivo", NUCLEIC ACIDS RESEARCH, vol. 45, no. 13, 7 June 2017 (2017-06-07), pages 7581 - 7592, XP055599779, ISSN: 0305-1048, DOI: 10.1093/nar/gkx507
- LAU SHANNEN ET AL: "Enhanced Extravasation, Stability and in Vivo Cardiac Gene Silencing via in Situ siRNA-Albumin Conjugation", MOLECULAR PHARMACEUTICS, vol. 9, no. 1, 5 December 2012 (2012-12-05), pages 71 - 80, XP008164358, ISSN: 1543-8384, [retrieved on 20111117], DOI: 10.1021/MP200246B
- TAI WANYI; LI JUNWEI; COREY EVA; GAO XIAOHU: "A ribonucleoprotein octamer for targeted siRNA delivery", NATURE BIOMEDICAL ENGINEERING, vol. 2, no. 5, 2 April 2018 (2018-04-02), London , pages 326 - 337, XP036513430, DOI: 10.1038/s41551-018-0214-1
- DERKS YVONNE H.W., LÖWIK DENNIS W. P. M., SEDELAAR J. P. MICHIEL, GOTTHARDT MARTIN, BOERMAN OTTO C., RIJPKEMA MARK, LÜTJE SUSANNE,: "PSMA-targeting agents for radio- and fluorescence-guided prostate cancer surgery", THERANOSTICS, vol. 9, no. 23, 1 January 2019 (2019-01-01), AU , pages 6824 - 6839, XP055977969, ISSN: 1838-7640, DOI: 10.7150/thno.36739

## Description

### CROSS-REFERENCE TO RELATED APPLICATIONS

This application claims the benefit of Chinese patent application No.202110442394.6, filed April 23, 2021, which is incorporated by reference as if fully set forth.

### FIELD OF INVENTION

The present disclosure relates to the field of biotechnology, in particular to a conjugate, preparation method therefor and use thereof.

### BACKGROUND

Prostate specific membrane antigen (PSMA), also known as folic hydrolase 1 (FOLH1), glutamic acid carboxypeptidase II (GCP II), N-acetyl-α-linked acid dipeptidase I (NAALAD1), belongs to the M28B subfamily of the peptidase M28 family. PSMA is a type II transmembrane protein with a homodimer structure,which is capable of binding two zinc ions per subunit. PSMA has both folate hydrolase and N-acetyl-α-linked acid dipeptidase (NAALADase) activity, which regulates the hydrolysis of N-acyl polyγ-glutamic acid and derivatives thereof. Compared with other tissues, PSMA is highly expressed in the nervous system, prostate, pancreas, kidney and small intestine, and its expression is much higher in the epithelial layer of most prostate cancers and in the neovascularization of other solid tumors than in normal tissues. This tissue specificity makes PSMA one of the most attractive targets for prostate cancer delivery.

Due to the high enrichment of PSMA in prostate cancer cells, antibodies, aptamers and small molecule ligands that specifically recognize PSMA are scrambling for research and development. The study of small molecule ligands for PSMA has been of great interest, due to the simplicity of synthesis and high protein binding affinity. As a small molecule ligand of PSMA, lysine/glutamic acid urea-linked dipeptide (KUE) has been successfully applied in prostate cancer imaging and targeted therapy. In addition, RNA interference (RNAi) is a novel technology that uses therapeutic oligonucleotides such as siRNAs to treat various diseases, including cancer. Encouraged by the approvals of Patisiran and Givosiran by Food and Drug Administration (FDA), RNAi is also gaining attention for the treatment of prostate cancer. Moreover, the approval of Givosiran makes the direct conjugate of a ligand to a siRNA a simple and effective new way for targeted cancer therapy. *
- Wanyi Tai et al., 2018, Advanced Biosystems, DOI: 10.1002/ADBI.201800075 relates to synthetic polymer tag for intracellular delivery of SiRNA providing neutral surface charge and endosomolytic capability with low immune response and easy to make. WO 2012/030745 relates to a method for subsequently labeling ligand molecules via Click chemistry by introducing propargyl-containing phosphoramidite monomers at either the 5' or 3' end of oligonucleotides. WO 2018/076025 relates to a molecular nanotag.

However, how to effectively deliver therapeutic siRNA into prostate cancer tissues/cells to achieve cancer treatment remains a significant challenge. Additionally, how to break through the limitations of the solid-phase synthesis method used by Givosiran (such as poor flexibility, complicated operation, unsatisfactory synthesis efficiency, and narrow application range and so on) is also a key problem that needs to be solved urgently.

### SUMMARY

The object of the present disclosure is to overcome the technical problems such as poor flexibility, complicated operation, and limited production scale of production of nucleic acid conjugates existing in the prior art, and to provide a conjugate, preparation method therefor and use thereof. The conjugate is characterized by both of high targeting and high activity. The method provided by the disclosure has the advantages of rapid, efficient, simple and widely applicable as well. In addition, the present disclosure provides the use of nucleic acid conjugates prepared according to the present disclosure in the preparation of a drug for the treatment of any one of prostate cancer, colon cancer, pancreatic cancer, breast cancer, kidney disease, and nervous system related diseases, in particular the drug for the treatment of prostate cancer.

To achieve the above objects, the first aspect of the present invention provides a conjugate formed by an azido-modified targeting ligand covalently linked to a propargyl siRNA sequence according to claim 1.

The second aspect of the present invention provides a method for preparing the aforementioned conjugate, comprising making contact between an azido-modified targeting ligand and a propargyl-modified siRNA sequence in the presence of a copper monovalent catalyst according to claim 4.

The third aspect of the present invention provides an use of the aforementioned conjugate or a conjugate prepared by the aforementioned method in the preparation of a drug for the treatment of a disease related to an abnormality in a tissue expressing PSMA, preferably occurring in glandular tissue, colon, kidney, and nervous system; wherein the glandular tissue is selected from one of prostate, pancreas, breast and thymus according to claim 9.

The present disclosure enables efficient and rapid construction of nucleic acid conjugates of the present disclosure through the ingenious design of propargyl-modified oligonucleotides (especially modified at the 3' -end) and azido-modified targeting ligands, and by utilizing the method of the present disclosure, namely post-synthesis modification strategy (copper-catalyzed click chemistry). Through cell imaging, gene silencing evaluation, and apoptosis experiments, it is found that the conjugate (nucleic acid conjugate) provided by the disclosure has high specific recognition performance, gene silencing performance, and inhibition of tumor cell generation performance. The method for preparing conjugates (nucleic acid conjugates) provided by the present disclosure has broad application prospects in targeted delivery of RNAi therapeutic reagents and therapy. In addition, the preparation method provided by the present disclosure only involves simple chemical reactions, but it can achieve flexible and efficient synthesis of nucleic acid conjugates, and is also applicable to the construction of a wild variety of other ligand-targeted nucleic acid conjugates, which is of good practicability.

### BRIEF DESCRIPTION OF THE DRAWINGS

FIG. 1 is a schematic diagram of a KUE-conjugated small nucleic acid in the present disclosure;
FIG. 2 is a high performance liquid chromatography chromatogram and a mass spectrum of 3'-terminal propargyl-modified oligonucleotide 1 (ON1) in the present disclosure;
FIG. 3 is a high performance liquid chromatography chromatogram and a mass spectrum of 3'-terminal propargyl-modified oligonucleotide 2 (ON2) in the present disclosure;
FIG. 4 is a high performance liquid chromatography chromatogram and a mass spectrum of 3'-terminal propargyl-modified oligonucleotide 3 (ON3) in the present disclosure;
FIG. 5 is a polyacrylamide gel electrophoresis characterization of different KUE-PEG-siRNA conjugates in the present disclosure;
FIG. 6 is a polyacrylamide gel electrophoresis characterization of different DUPA-PEG₅-siRNA conjugates in the present disclosure;
FIG. 7 is a mass spectrum of the nucleic acid conjugate KUE-PEG₂-siRNA in Example 1 of the present disclosure;
FIG. 8 is a mass spectrum of the nucleic acid conjugate KUE-PEG₅-siRNA in Example 21 of the present disclosure;
FIG. 9 is a mass spectrum of the nucleic acid conjugate KUE-PEG₁₂-siRNA in Example 22 of the present disclosure;
FIG. 10 is a mass spectrum of the nucleic acid conjugate DUPA-PEG₅-siRNA in Example 23 of the present disclosure;
FIG. 11 is a mass spectrum of the nucleic acid conjugate DUPA-PEG₅-siRNA in Example 24 of the present disclosure;
FIG. 12 is a diagram which shows the cell imaging results of different nucleic acid conjugates in one specific embodiment of the present disclosure;
FIG. 13 is a statistical chart of the mean intracellular fluorescence intensity of different nucleic acid conjugates in one specific embodiment of the present disclosure;
FIG. 14 is a diagram which shows the results of evaluation of the cellular internalization mode of different nucleic acid conjugates in one specific embodiment of the present disclosure;
FIG. 15 is an evaluation diagram of uptake efficiency of different nucleic acid conjugates in prostate cancer cells in one specific embodiment of the present disclosure;
FIG. 16 is a diagram of mRNA silencing results of different nucleic acid conjugates in one specific embodiment of the present disclosure;
FIG. 17 is a diagram of protein expression inhibition results of different nucleic acid conjugates in one specific embodiment of the present disclosure;
FIG. 18 is a diagram shows the results of apoptosis promoted by different nucleic acid conjugates in the present disclosure.

### DETAILED DESCRIPTION OF THE INVENTION

The end points and any values of the ranges disclosed herein are not limited to that precise range or value, which should be understood to include values close to those ranges or values. "For numerical ranges, one or more new numerical ranges may be combined between the endpoint values of the individual ranges, between the endpoint values of the individual ranges and individual point values, and between the individual point values, which shall be deemed to be specifically disclosed herein.

The inventors found that the azido modified targeting ligands (such as azido modified KUE ligand, azido modified DUPA ligand, etc.) and 3' -terminal propargyl modified small nucleic acid (oligonucleotide) can flexibly and efficiently construct antigen (such as PSMA) targeted siRNA conjugates with a huge number of studies. In addition, in order to verify whether 3'-terminal-propargyl-modified small nucleic acids can maintain their thermal stability, overall conformation and RNAi activity, the inventors studied the related properties of 3'-terminal-propargyl-modified small nucleic acids in aspects such as melting chain temperature, conformation and RNAi activity evaluation, and found that alkyne-modification of 3'-terminal of siRNAs not only does not affect the intrinsic properties of the RNAs, but also accentuates the properties of the post-synthesis modifications of the siRNAs.

Based on the context above, the first aspect of the present invention provides a conjugate formed by an azido-modified targeting ligand covalently linked to a propargyl-modified siRNA sequence according to claim 1.

According to some embodiments of the present disclosure, the azide group in the azido-modified targeting ligand is covalently linked to the targeting ligand by at least one fragment of polyethylene glycol. Wherein, the number of PEG monomers in the PEG fragment is not specially limited, for example, it can be 1-100. Wherein, the fragment of the PEG mainly functions as a linker.

According to some embodiments of the present disclosure, the targeting ligand is a dipeptide formed by two amino acids in which at least one amino acid is glutamic acid; preferably, the amino acids are selected from lysine and glutamic acid, glutamic acid and glutamic acid, and glutamic acid and glutamate analogs; wherein, the targeting ligand is able to recognize and bind to antigens expressed on the surface of target cells through glutamate; The antigen is PSMA.

According to some embodiments of the present disclosure, the end of the targeting ligand contains a carboxyl group; the targeting ligand is linked by a carboxyl group to an antigen on the surface of the target cell.

According to some embodiments of the present disclosure, the target cell may be selected from at least one of prostate cancer cells, neuronal cells, kidney cancer cells, and colon cancer cells.

According to the present invention the azido modified targeting ligand has a structure as follows: 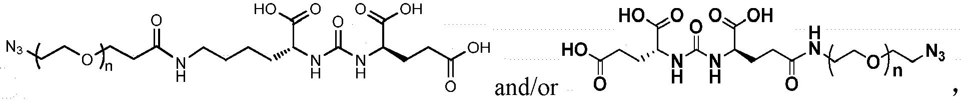 wherein n is an integer from 1 to 100. Preferably, n is an integer from 1 to 50, more preferably an integer from 1 to 20, further preferably an integer from 1 to 15. For example, it could be 1, 2, 3, 4, 5, 6, 7, 8, 9, 10, 11, 12, 13, 14, 15.

In the present disclosure, the azide-modified targeting ligand can be prepared by the following method:
Firstly, KUE was synthesized by linking lysine protected by t-butyl ester and benzyl ester with glutamate protected by t-butyl ester through triphosgene; After that, the ethylene glycol was acrylated and azided to obtain azido PEG propionic acid; Finally, the targeting ligand can be obtained by condensation reaction with both of them (KUE and azido PEG propionic acid).

In the present disclosure, preferably, the azide-modified targeting ligand (**6a**(n=2), **6b**(n=5), 6c(n=12)) can be prepared by the following steps:
(1) The polyethylene glycol **1a-c** was exposed to the metal sodium, followed by the first reaction with tert-butyl acrylate to obtain the compound shown in **2a-c;** 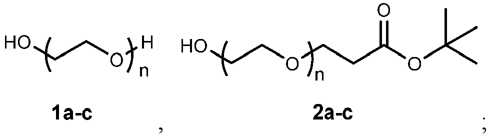
(2) In the presence of triethylamine, the compound shown in Formula **2a-c** was subjected to a second reaction with tosyl chloride to obtain the compound shown in Formula **3a-c;** 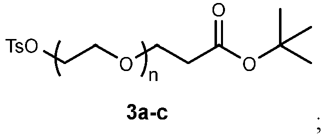
(3) In the presence of the third solvent, the compound shown in Formula **3a-c** was subjected to the third reaction with sodium azide to obtain the compound shown in Formula **4a-c;** 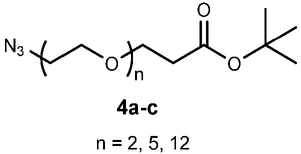
(4) In the presence of HATU (2-(7-aza-benzotriazole)-N,N,N',N'-tetramethylurea hexafluorophosphate) and DIPEA (N,N-diisopropylethylamine), the compound shown in **4a-c** was subjected to the fourth reaction with KUE to obtain the compound shown in Formula **5a-c;** 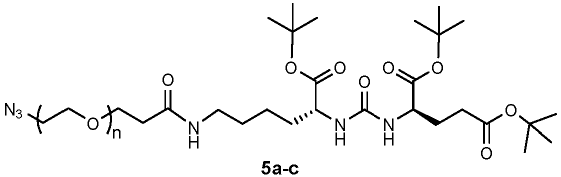
(5) The compound shown in Formula **5a-c** was subjected to the fifth reaction with trifluoroacetic acid to obtain the compound shown in Formula **6a-c;**

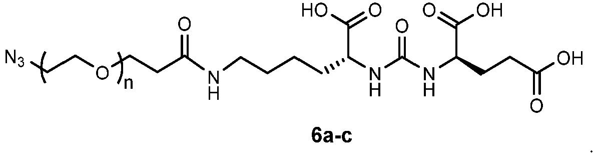

In step (1), the reaction temperature of the first reaction may be 20-40°C, and the reaction time of the first reaction may be 5-20 hours. Wherein, relative to each millimole of polyethylene glycol (**1a-c**), the amount of metal sodium may be 0.05-2mmol, the amount of t-butyl acrylate may be 0.3-0.8mmol. The first reaction is carried out in the presence of a first solvent; Wherein, relative to each millimole of polyethylene glycol, the amount of the first solvent may be 1-2mL; The first solvent may be tetrahydrofuran.

In step (2), the reaction temperature of the second reaction may be from -20°C to 10°C, and the reaction time of the second reaction may be 5-20 hours. Wherein, relative to each millimole of the compound shown in Formula **2a-c,** the amount of triethylamine is 0.5-1ml, and the amount of tosyl chloride may be 1.2-1.8mmol. The second reaction is carried out in the presence of a second solvent; wherein, relative to each millimole of the compound shown in Formula **2a-c,** the amount of the second solvent may be 1-3mL. Wherein, the second solvent may be dichloromethane. The second reaction is preferably carried out under an inert atmosphere, which may be provided by argon.

In step (3), the reaction temperature of the third reaction may be 60-90°C and the reaction time of the third reaction may be 5-24 hours. Wherein, relative to each millimole of the compound shown in Formula **3a-c,** the amount of sodium azide may be 1.2-1.8mmol. Wherein, relative to each millimole of the compound shown in Formula **3a-c,** the amount of the third solvent may be 2-3mL. The third solvent may be DMF (N,N dimethylformamide).

In step (4), the reaction temperature of the fourth reaction may be 20-40°C and the reaction time of the fourth reaction may be 5-24 hours. Wherein, relative to each millimole of the compound shown in Formula **4a-c,** the amount of KUE may be 0.9-1.5mmol. Wherein, prior to the fourth reaction between the compound shown in **4a-c** and KUE, the compound shown in **4a-c** may be deprotected in the presence of trifluoroacetic acid (the amount of trifluoroacetic acid is 2-3ml relative to 1 mol of the compound shown in **4a-c**), wherein the temperature of de-protection may be 20-30°C, and the time of de-protection is 10-24h; HATU and DIPEA may then be used to activate the de-protection products of the compound shown in **4a-c;** Wherein, relative to each millimole of the compound shown in Formula **4a-c,** the amount of HATU and DIPEA may be 1.2-1.8mmol and 2-4mmol, respectively. The fourth reaction is carried out in the presence of a fourth solvent; Wherein, relative to each millimole of the compound shown in Formula **4a-c,** the amount of the fourth solvent may be 0.8-1.5mL. The fourth solvent may be DMF (N,N dimethylformamide).

In step (5), the reaction temperature of the fifth reaction may be 20-40°C and the reaction time of the fifth reaction may be 5-24 hours. Wherein, relative to each millimole of the compound shown in Formula **5a-c,** the amount of trifluoroacetic acid may be 20-30mL.

Wherein, there is no special restriction on the post-treatment of the reactions in steps (1) - (5), and all the purified products may be obtained by column chromatography separation.

In the present disclosure, the structural formula of the KUE is as follows:

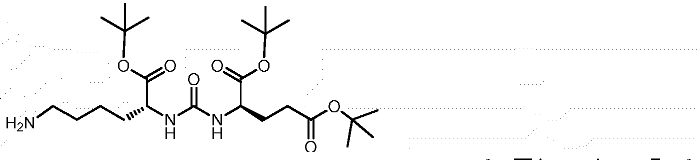

According to some embodiments of the present invention the small nucleic acid sequence may be selected from at least one of the siRNAs targeting STAT3, PHB1, Notch1, PLK1, and BRD4.

According to a preferred embodiment of the present invention the propargyl modification is preferably a 3'-terminal propargyl modification.

According to some embodiments of the present invention, the alkyne-modified small nucleic acid sequence is modified to the 3'-terminal of the siRNA acid sequence using the phosphoramidite method by solid-phase synthesis techniques with propargyl compound; Wherein, the propargyl compound contains at least one active hydroxyl group. Wherein, the propargyl modification may be more than one propargyl modifications (1, 2, 3...). In the present disclosure, there is no particular limitation on the manner in which the propargyl compound is modified to the 3'-end of the siRNA sequence, it may be performed as follows: First, based on the prior art (Bioorg. Med. Chem., 2013, 5583-5588), 1-O-propargyl-2-deoxy-D-furanose phosphoramidite monomer was synthesized from commercial Hoffer's chlorosugar through four steps. Then, the resulting phosphoramidite monomer was linked to a controlled pore glass (CPG) and the 3'-terminal of the propargyl monomer X oligonucleotide was inserted by an automatic nucleic acid synthesizer; Finally, the CPG and protective group were removed to obtain 3'-terminal propargyl-modified siRNA acids.

In the present invention the propargyl compound has a structure as follows: 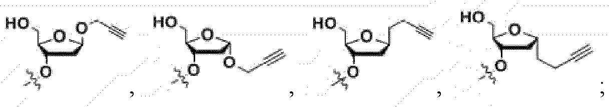 wherein " 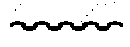 " represents the siRNA ligation site.

The second aspect of the present disclosure provides a method for preparing the aforementioned conjugate, comprising making contact between an azido-modified targeting ligand and a propargyl-modified siRNA sequence in the presence of a copper monovalent catalyst.

According to some embodiments of the present disclosure, the molar ratio of the azido-modified targeting ligand to the propargyl-modified siRNA sequence may be (1.05-10) : 1, preferably (2-4) : 1.

According to some embodiments of the present disclosure, the copper monovalent catalyst may be selected from at least one of Cu(I)-TBTA, CuBr, and CuCl, preferably Cu(I)-TBTA.

According to some embodiments of the present disclosure, relative to 1 mol of the siRNA sequence, the amount of the copper monovalent catalyst may be 2-10 mol, preferably 3-6 mol,.

According to some embodiments of the present disclosure, the conditions of the contact comprising: temperature may be 35.5-38.5 °C, preferably 36.5-37.5 °C; the time of the contact may be 1-5h, preferably 2-4h.

In the present disclosure, there is no special limitation to the speed of the contact, as long as it can meet the requirements of the present disclosure, for example, the speed may be 600-1000r/min, preferably 700-900r/min.

In the present disclosure, the contact is preferably carried out under the condition of shock, and the condition of shock may be: once every 0.4-0.6h.

According to some embodiments of the present disclosure, the contact is carried out in the presence of an organic solvent selected from at least one of N,N-dimethylformamide (DMF), methanol, ethanol, and acetone.

According to some embodiments of the present disclosure, the amount of organic solvent is such that the concentration of the siRNA sequence is maintained between 20 and 200µM, preferably between 80 and 120µM.

In the present disclosure, the conjugate may refer to a single-stranded nucleic acid conjugate or a double-stranded nucleic acid conjugate; When the conjugate is a double-stranded nucleic acid conjugate, it may be formed by annealing from the sense and antisense strand nucleic acids with reference to conventional means in the art, for example, by forming the sense and antisense strand nucleic acids in equimolar proportions in an annealing buffer (containing magnesium ions) at 90-100 °C.

According to some embodiments of the present disclosure, the siRNA sequence comprises a sense strand and an antisense strand, and the method comprising:
(1) Propargyl-modified sense strand and propargyl-modified antisense strand were obtained seperately;
(2) Single-stranded conjugate was obtained by contacting the azide-modified target ligand with the propargyl-modified sense strand or propargyl-modified antisense strand;
(3) The conjugate was obtained by incubating the single-stranded conjugate with the other strand in annealing buffer at 90-100 °C for 1-5 minutes;

According to a preferred embodiment of the present disclosure, the annealing buffer is selected from a magnesium acetate solution of 1.5-2.5mM.

The third aspect of the disclosure provides an use of the aforementioned conjugate or a conjugate prepared by the aforementioned method in the preparation of a drug for the treatment of a disease related to an abnormality in a tissue expressing PSMA, preferably occurring in glandular tissue, colon, kidney, and nervous system; wherein, the glandular tissue is selected from one of the prostate, pancreas, breast and thymus. wherein, the disease is preferably a cancer.

In the present disclosure, "conjugate" refers to "KUE-PEG-siRNA conjugate" or "DUPA-PEG-siRNA conjugate".

The present disclosure will be described in detail by examples below.

In the following examples, all raw materials are commercially available.

### Preparation Example 1

This preparation example is used to illustrate the preparation of azide-modified KUE ligand 6a-**c.**

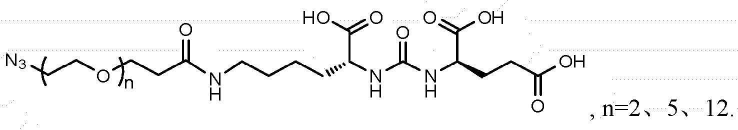

### Synthetic route 1:

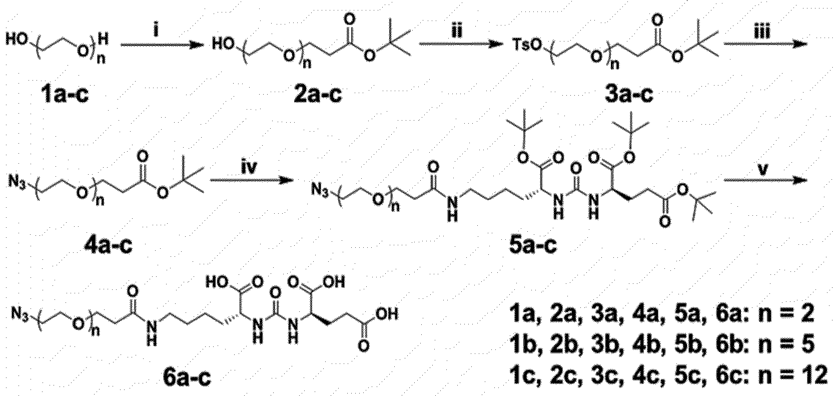

### Specific operation steps:

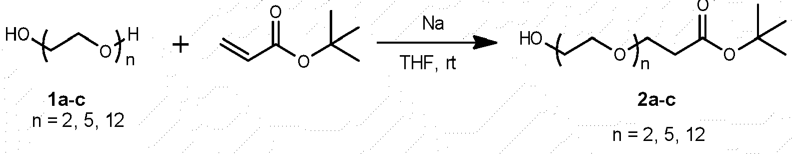

(i) Polyethylene glycosl **1a-c** (100mmol, 1eq) were dissolved in dry THF (150mL), to which metal sodium (230mg, 10mmol, 0.1eq) was added and stirred at room temperature until the sodium was completely dissolved. Subsequently, tert-butyl acrylate (6.4g, 50mmol, 0.5eq) was added slowly to the mixture and reacted for 12 hours at room temperature. At the end of the reaction, the reaction was quenched by the addition of 8mL of 1M aqueous hydrochloric acid. After stirring for 10 minutes, the reaction solution was poured into 200mL of saturated saline, extracted with ethyl acetate for 3 times, the organic phase was collected, washed with saturated saline, dried with anhydrous sodium sulfate, and purified by rapid column chromatography (PE/EA=10:1-1:1) to obtain a slightly yellow mono-tert-bytyl ester product. 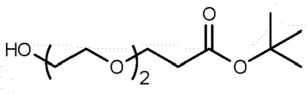 ¹H-NMR (400 MHz, CDCl₃) δ 3.77 - 3.70 (m, 4H), 3.69 - 3.59 (m, 6H), 2.78 (s, 1H), 2.54 - 2.49 (m, 2H), 1.45 (s, 9H); ¹³C-NMR (101 MHz, CDCl₃) δ170.92, 80.63, 72.49, 70.33, 70.31, 66.81, 61.70, 36.14, 28.05. HRMS: calculated for C₁₁H₂₂NaO₅ [M+Na]⁺: 257.1365, found 257.1357.

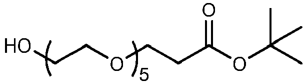

¹H-NMR (400 MHz, CDCl₃) δ 3.79 - 3.59 (m, 22H), 2.91 (s, 1H), 2.53 - 2.48 (m, 2H), 1.45 (s, 9H); ¹³C-NMR (101 MHz, CDCl₃) δ 170.93, 80.51, 72.52, 70.61, 70.59, 70.56, 70.54, 70.48, 70.33, 66.88, 61.72, 36.24, 28.09. HRMS: calculated for C₁₇H₃₄NaO₈ [M+Na]⁺: 389.2151, found 389.2146.

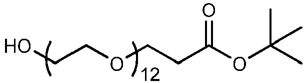

¹H-NMR (400 MHz, CDCl₃) δ 3.76 - 3.54 (m, 49H), 2.77 (s, 2H), 2.53 - 2.48 (m, 2H), 1.45 (d, J = 2.4 Hz, 9H); ¹³C-NMR (101 MHz, CDCl₃) δ 170.94, 80.53, 72.58, 70.61, 70.57, 70.51, 70.37, 70.29, 66.90, 61.71, 36.26, 28.10. HRMS: calculated for C₃₁H₆₂NaO₁₅ [M+Na]⁺: 697.3986, found 697.3981.

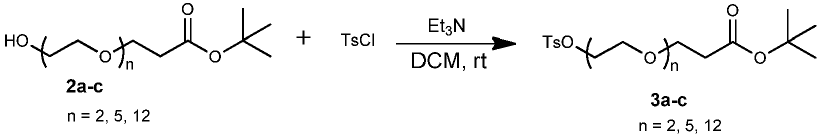

(ii) Compound **2a-c** (12.3mmol, 1eq) was dissolved in a mixture of 30mL dichloromethane and 10mL triethylamine and stirred in an ice bath under an argon atmosphere. Then, p-toluenesulfonyl chloride (3.53g, 18.5mmol, 1.5eq) was added in batches. The mixture was stirred overnight at room temperature, then, 10g of a small amount of silica gel (100-200 mesh) was concentrated to dryness, and chromatographic separation (PE/EA=10:1-1:3) was performed on silica gel (300-400 mesh), obtaining a colorless oil form product.

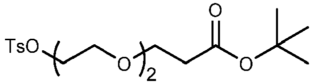

¹H-NMR (400 MHz, CDCl₃) δ 7.79 (d, *J* = 8.4 Hz, 2H), 7.35 (d, *J* = 8.0 Hz, 2H), 4.19 - 4.11 (m, 2H), 3.67 (dd, *J* = 8.0, 4.8 Hz, 4H), 3.55 (q, *J* = 4.8 Hz, 4H), 2.47 (dd, *J* = 12.4, 6.0 Hz, 5H), 1.44 (s, 9H); ¹³C-NMR (101 MHz, CDCl₃) δ 170.81, 144.80, 132.99, 129.82, 127.95, 80.50, 70.60, 70.29, 69.26, 68.64, 66.88, 36.21, 28.07, 21.61. HRMS: calculated for C₁₈H₂₈NaO₇S [M+Na]⁺: 411.1453, found 411.1448.

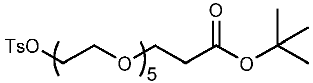

¹H-NMR (400 MHz, CDCl₃) δ 7.85 - 7.74 (m, 2H), 7.37 - 7.33 (m, 2H), 4.24 - 4.08 (m, 2H), 3.83 - 3.51 (m, 20H), 2.59 - 2.36 (m, 5H), 1.45 (d, *J =* 5.6 Hz, 9H); ¹³C-NMR (101 MHz, CDCl₃) δ 170.89, 144.78, 133.02, 129.82, 127.98, 80.49, 70.74, 70.58, 70.51, 70.36, 69.24, 68.67, 66.89, 36.27, 28.09, 21.64. HRMS: calculated for C₂₄H₄NaO₁₀S [M+Na]⁺: 543.2240, found 543.2246.

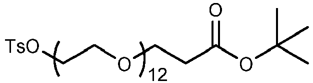

¹H-NMR (400 MHz, DMSO) δ 7.83 - 7.74 (m, 2H), 7.49 (d, J = 7.6 Hz, 2H), 4.13 - 4.10 (m, 2H), 3.64 - 3.29 (m, 48H), 2.43 (s, 5H), 1.40 (s, 9H); ¹³C-NMR (101 MHz, DMSO) δ 170.87, 145.34, 132.88, 130.59, 128.10, 80.15, 70.44, 70.25, 70.17, 70.14, 68.35, 66.69, 36.29, 28.20, 21.55. HRMS: calculated for C₃₈H₆₈NaO₁₇S [M+Na]⁺: 851.4075, found 851.4071.

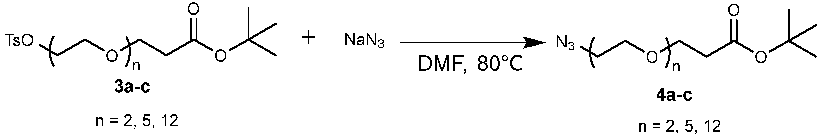

(iii) Compound **3a-c** (2mmol, 1eq) was dissolved in 5mL of dimethylformamide and sodium azide (195mg, 3mmol, 1.5eq) was added. The mixture was allowed to react overnight at 80°C, and then concentrated to dryness by adding 2g of a small amount of silica gel (100-200 mesh) and chromatographic separation (PE/EA=20:1-1:2) was performed on silica gel (300-400 mesh), resulting in a colorless oil form product.

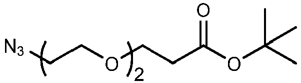

¹H-NMR (400 MHz, CDCl₃) δ 3.76 - 3.60 (m, 8H), 3.42 - 3.35 (m, 2H), 2.51 (t, *J* = 6.4 Hz, 2H), 1.45 (s, 9H); ¹³C-NMR (101 MHz, CDCl₃) δ 170.84, 80.45, 70.56, 70.38, 70.00, 66.92, 50.64, 36.22, 28.04. HRMS: calculated for C₁₁H₂₁N₃NaO₄ [M+Na]⁺: 282.1430, found 282.1426.

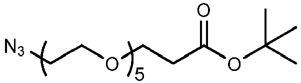

¹H-NMR (400 MHz, CDCl₃) δ 3.77 - 3.58 (m, 20H), 3.47 - 3.30 (m, 2H), 2.50 (t, *J =* 6.8 Hz, 2H), 1.45 (s, 9H); ¹³C-NMR (101 MHz, CDCl₃) δ 170.92, 80.51, 70.70, 70.67, 70.64, 70.59, 70.50, 70.37, 70.04, 66.90, 50.68, 36.26, 28.09. HRMS: calculated for C₁₇H₃₃N₃NaO₇ [M+Na]⁺: 414.2216, found 414.2211.

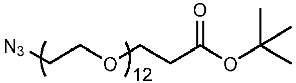

¹H-NMR (400 MHz, CDCl₃) δ 3.75 - 3.57 (m, 48H), 3.40 (d, *J =* 4.4 Hz, 2H), 2.53 - 2.48 (m, 2H), 1.45 (d, J = 2.4 Hz, 9H); ¹³C-NMR (101 MHz, CDCl₃) δ 170.91, 80.49, 70.69, 70.66, 70.63, 70.56, 70.49, 70.36, 70.04, 66.88, 50.67, 36.25, 28.09. HRMS: calculated for C₃₁H₆₁N₃NaO₄ [M+Na]⁺: 722.4051, found 722.4048.

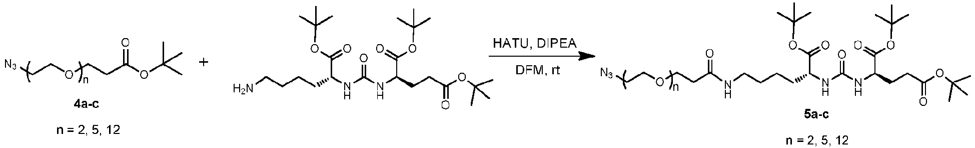

(iv) Compound **4a-c** (2mmol, 1eq) was dissolved in 5mL of trifluoroacetic acid and was allowed to react overnight at room temperature. Then, the remaining trifluoroacetic acid was removed by concentration, dichloromethane and 2g silica gel (100-200 mesh) were added and concentrated to dryness, and chromatographic separation (PE/EA=5:1-1:5) was carried out on silica gel (300-400 mesh), resulting a product as colorless oil, which was dissolved in 2mL of dimethylformamide. HATU (1.15g, 3mmol, 1.5eq) and DIPEA (775.5mg, 6mmol, 3eq) were added for activation. After 15min, KUE (1.17g, 2.4mmol, 1.2eq) was added. The mixture was allowed to react overnight at room temperature, then, 3g of small amount of silica gel (100-200 mesh) was concentrated to dryness, and chromatographic separation (PE/EA=10:1-1:5) was performed on silica gel (300-400 mesh), obtaining the product in colorless oil form.

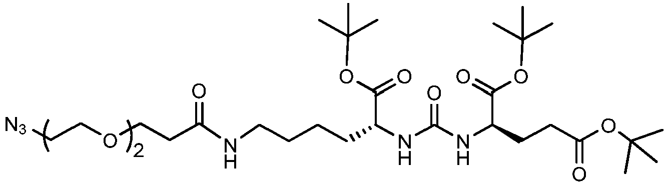

¹H-NMR (400 MHz, CDCl₃) δ 6.87 (s, 1H), 5.64 (dd, J = 20.0, 6.4 Hz, 2H), 4.38 - 4.17 (m, 2H), 3.71 (d, J = 28.0 Hz, 7H), 3.44 (s, 2H), 3.34 - 3.12 (m, 2H), 2.52 (s, 2H), 2.32 (d, J = 5.2 Hz, 2H), 2.06 (d, J = 6.0 Hz, 1H), 1.84 (dd, J = 32.0, 25.2 Hz, 2H), 1.67 - 1.23 (m, 33H); ¹³C-NMR (101 MHz, CDCl₃) δ 172.58, 172.52, 157.40, 82.17, 81.66, 80.67, 70.19, 70.00, 69.76, 67.35, 53.35, 52.99, 50.56, 38.84, 36.42, 32.02, 31.55, 28.63, 28.04, 27.96, 22.23. HRMS: calculated for C₃₁H₅₆N₆NaO₁₀ [M+Na]⁺: 695.3956, found 695.3953.

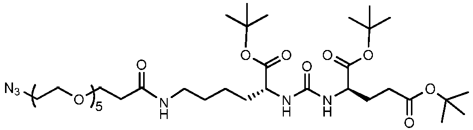

¹H-NMR (400 MHz, CDCl₃) δ 7.05 (s, 1H), 5.78 (dd, J *=* 16.4, 8.0 Hz, 2H), 4.38 - 4.17 (m, 2H), 3.71 (d, J = 29.2 Hz, 15H), 3.41 (d, J = 4.0 Hz, 1H), 3.26 (dd, J = 23.2, 17.2 Hz, 2H), 3.00 (s, 3H), 2.50 (s, 1H), 2.32 (d, J = 6.4 Hz, 2H), 2.05 (s, 1H), 1.91 - 1.70 (m, 2H), 1.69 - 1.17 (m, 36H); ¹³C-NMR (101 MHz, CDCl₃) δ 172.21, 161.56, 157.58, 157.41, 81.74, 81.19, 80.34, 70.47, 70.45, 70.41, 70.37, 70.35, 70.28, 70.18, 70.01, 69.87, 67.24, 53.35, 52.83, 50.54, 45.11, 39.74, 38.74, 36.60, 32.17, 31.92, 31.47, 29.03, 28.70, 28.20, 27.97, 27.91, 22.55, 22.31. HRMS: calculated for C₃₇H₆₈N₆NaO₁₃ [M+Na]⁺: 827.4742, found 827.4738.

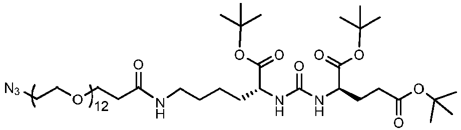

¹H-NMR (400 MHz, CDCl₃) δ 6.89 (s, 1H), 5.57 (s, 2H), 4.26 (d, J = 20.4 Hz, 2H), 3.67 (s, 45H), 3.41 (s, 2H), 3.21 (d, J = 16.8 Hz, 2H), 2.99 (s, 1H), 2.53 (s, 1H), 2.32 (s, 2H), 2.05 (s, 1H), 1.80 (d, J = 44.4 Hz, 1H), 1.65 - 1.18 (m, 36H); ¹³C-NMR (101 MHz, CDCl₃) δ 172.41, 157.42, 81.87, 81.44, 80.50, 70.37, 70.21, 70.05, 69.99, 69.82, 69.71, 69.62, 69.49, 69.37, 53.47, 53.02, 50.61, 38.87, 31.97, 31.55, 28.20, 28.06, 27.98, 22.31. HRMS: calculated for C₅₁H₉₆N₆NaO₂₀ [M+Na]⁺: 1135.6577, found 1135.6569.

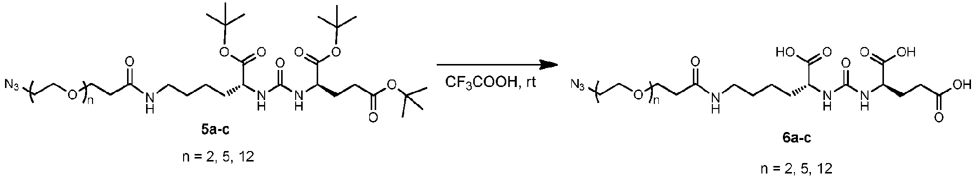

(v) Compound **5a-c** (0.2mmol, 1eq) was dissolved in 5mL of trifluoroacetic acid and was allowed to react overnight at room temperature. Then, the remaining trifluoroacetic acid was removed by concentration, dichloromethane and 1g of small amount of silica gel (100-200 mesh) were added and concentrated to dryness, and chromatographic separation (DCM/MeOH=10:1-1:2) was carried out on silica gel (300-400 mesh), obtaining the product in colorless oil form. **The compounds shown in 6a-c** were confirmed by high-resolution mass spectrometry.
**6a:** 145mg, Yield 96%, HRMS: calcd. for C₁₉H₃₂N₆O₁₀ [M-H]⁻: 503.2102; MALDI-TOF-MS: m/z 503.2105;
**6b:** 179mg, Yield 94%, HRMS: calcd. for C₂₅H₄₄N₆O₁₃ [M-H]⁻: 635.2888; MALDI-TOF-MS: m/z 635.2892;
**6c:** 260mg, Yield 92%, HRMS: calcd. for C₃₉H₇₂N₆O₂₀ [M-H]⁻: 943.4723; MALDI-TOF-MS: m/z 943.5601_{∘}

### Preparation Example 2

This preparation example is used to illustrate the preparation of azide-modified DUPA ligands

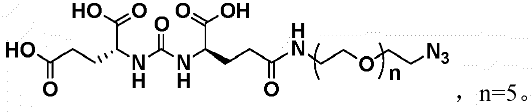

### Synthetic route 2:

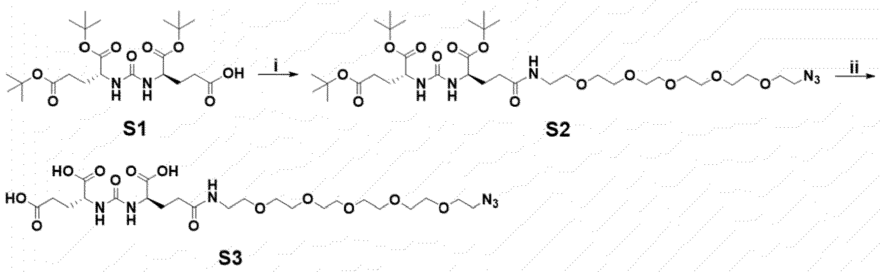

### Specific operation steps:

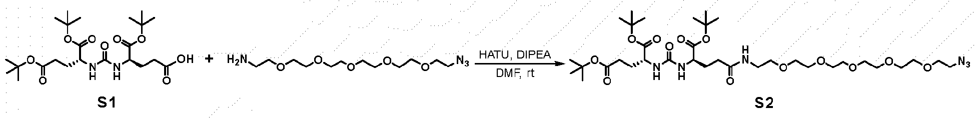

(i) Compound **S1** (244mg, 0.5mmol) and 2-(7-aza-benzotriazol-1-yl)-N,N,N',N' - tetramethylureinium hexafluorophosphate (HATU, 228 mg, 0.6 mmol) were added together into 5mL of dimethylformamide. After addition of N,N-diisopropyl ethylamine (DIPEA, 129 mg, 1 mmol) under stirring conditions, the mixture was stirred for 15 minutes at room temperature. 17-azido-3,6,9,12,15-pentaoxaheptadecan-1-amine (182mg, 0.6mmol) was immediately added to the mixture as a spray and the reaction was continued for 6 hours at room temperature. At the end of the reaction, the reaction mixture was poured into 20 mL of water, extracted with ethyl acetate (50mL×3), the organic phase was collected, washed with saturated salt water, dried with anhydrous sodium sulfate, and the solvent was removed under reduced pressure to obtain an orange oil crude product. The residue was separated and purified by column chromatography (ethyl acetate/petroleum ether =1/5) to obtain the yellow oil form compound **S2** (338 mg, yield: 87%).

¹H NMR (400 MHz, CDCl₃) δ6.93 <s, 1H) , 5.67 <s, 2H) , 4.28 <d, J = 7.3 Hz, 2H) , 3.66-3.50 (m, 20H) , 3.48-3.30 (m, 4H) , 2.30-1.96 (m, 6H) , 1.89-1.72 (m, 2H) , 1.47-1.29 (m, 27H) ; ¹³C NMR (101 MHz, CDCl₃)δ172.81, 172.56, 172.28, 171.95, 157.49, 81.89, 81.66, 80.44, 70.64, 70.62, 70.55, 70.52, 70.48, 70.43, 70.42, 70.05, 69.98, 69.49, 53.02, 50.63, 39.15, 32.54, 31.63, 29.19, 28.18, 28.05, 27.98. HRMS (ESI) : m/z [M+Na]⁺ calculated for C₃₅H₆₄N₆NaO₁₃⁺: 799.4424, found 799.4428.

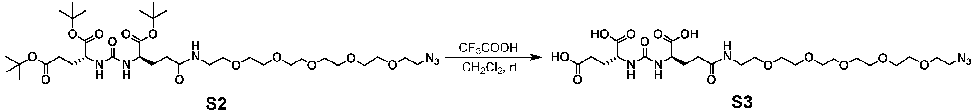

(ii) Compound **S2** (472 mg, 0.5 mmol) was dissolved in an equal volume mixed solution (5 mL) of dichloromethane and trifluoroacetic acid under ice bath conditions, and the reaction solution was left to react for 6 hours at room temperature. The solvent was removed by evaporation, and the resulting residue was purified by rapid column chromatography (MeOH/CH2Cl2=1/3) to obtain compound **S3** in oil form (238mg, 98%).

¹H NMR (400 MHz, DMSO-*d₆*) δ12.52 (s, 3H) , 7.94 (d, J = 9.2 Hz, 2H) , 6.36 (dd, J = 7.9, 5.6 Hz, 2H) , 4.16-3.98 (m, 2H) , 3.63-3.58 (m, 2H) , 3.57-3.50 (m, 14H) , 3.42-3.35 (m, 5H) , 3.18 (q, J = 5.9 Hz, 2H) , 2.29-2.19 (m, 2H) , 2.14-2.08 (m, 2H) , 1.95-1.84 (m, 2H) , 1.77-1.65 (m, 2H) ; ¹³C NMR (101 MHz, DMSO) δ174.77, 174.67, 174.27, 171.83, 162.78, 157.73, 70.28, 70.24, 70.16, 70.14, 69.72, 55.40, 52.58, 52.15, 50.43, 38.97, 36.25, 32.00, 31.23, 30.42, 28.71, 28.09. HRMS (ESI) : m/z [M-H]⁻calculated for C₂₃H₃₉N₆O₁₃⁻: 607.2581; found: 607.2578.

### Preparation Example 3

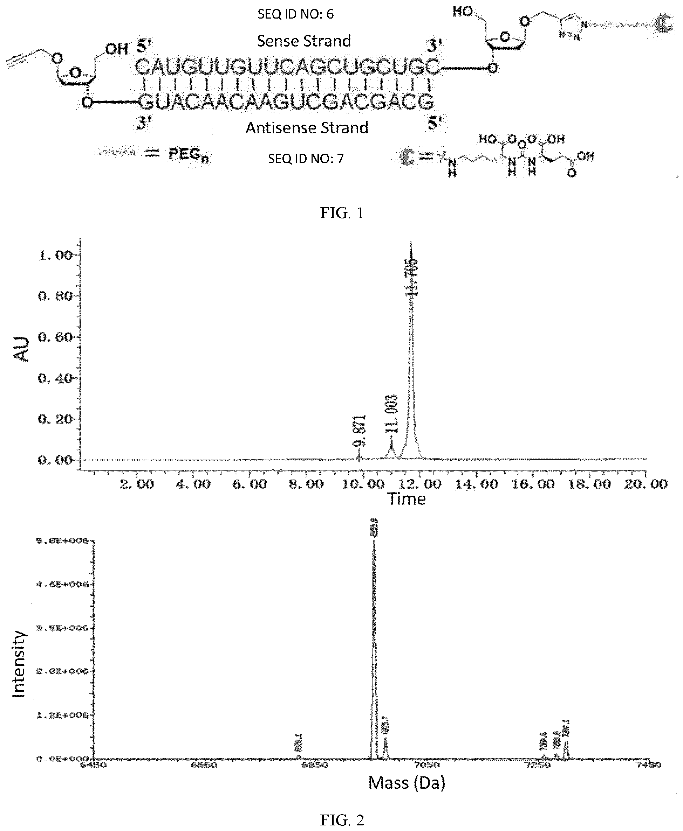

This preparation example is used to illustrate the synthesis of 3'-terminal propargyl-modified small nucleic acids
1-O-propargyl-2-deoxy-D-furanose (X, i.e. propargyl monomer), as a universal overhang modification, was attached to the 3'-terminal of small nucleic acids (oligonucleates) by using an ABI 394 DNA/RNA synthesizer (ABI, USA) (as shown in FIG. 1 for small nucleic acids of SEQ ID NO: 6 and 7).

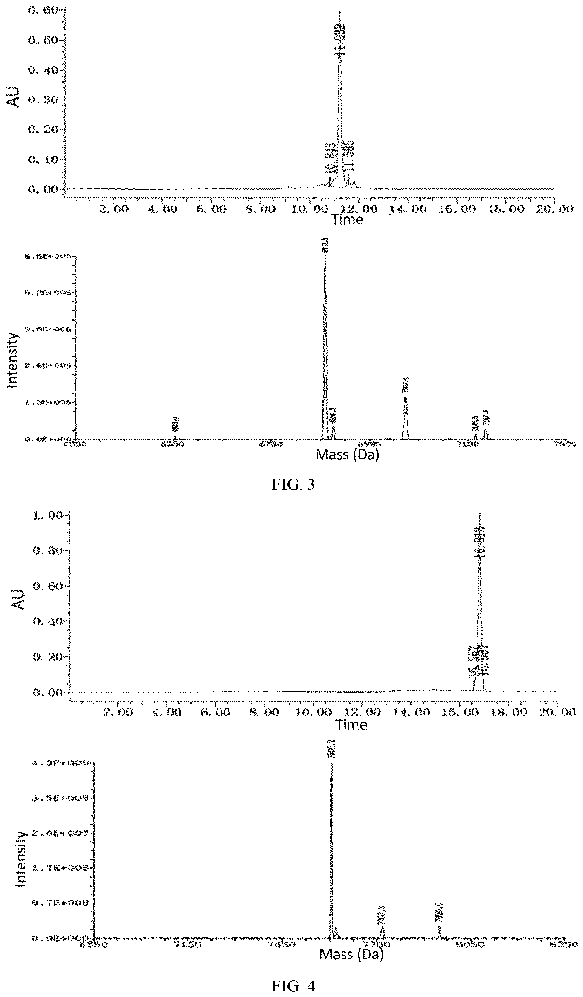

The specific steps of synthesis of 3'-terminal propargyl-modified small nucleic acids are as follows: firstly, 1-O-propargyl-2-deoxy-D-furanose phosphoramidite monomer was synthesized from commercial Hoffer's chlorosugar (Bioorg. Med. Chem., 2013, 5583-5588); then, the resulting phosphoramidite monomer was linked to a controlled pore glass (CPG), and linked to the 3'-terminal of the propargyl monomer X oligonucleotide by the automated nucleic acid synthesizer described above; finally, the CPG and the protective group were removed to obtain different 3'-terminal propargyl-modified single-stranded small nucleic acids. The purity of all propargyl-modified single-stranded nucleic acids prepared by the present disclosure was confirmed by high performance liquid chromatography and the structure was confirmed by mass spectrometer (FIG. 2-4).

**Table 1 Different 3'-terminal propargyl-modified single-stranded small nucleic acids**

| **ONs No.** | **Sequence (SS/AS)** | |
|---|---|---|
| ON**1** | 5'Cy5- CAUGUUGUUCAGCUGCUGCUUX -3' | SEQ ID NO: 1 |
| ON**2** | 5'- CAUGUUGUUCAGCUGCUGCUUX -3' | SEQ ID NO: 2 |
| ON**3** | 5'- GCAGCAGCUGAACAACAUGUUX -3' | SEQ ID NO: 3 |
| ON**4** | 5'- GCGACGACCUUACAGAGCGUUX -3' | SEQ ID NO: 4 |
| ON**5** | 5'- CGCUCUGUAAGGUCGUCGCUUX -3' | SEQ ID NO: 5 |

### Example 1

This example is used to illustrate the efficient synthesis of KUE-PEG-siRNA conjugates

Synthesis of KUE-PEG2-siRNA conjugates: Propargyl-modified small nucleic acid sequence (ON3) (10 nmol), azido-modified targeting ligand **6a** (30 nmol), and Cu(I)-TBTA (50nmol) were successively added into a 200µL sterile centrifugation tube to maintain the final concentration of RNA at about 100µM. DMF (25% of the reaction liquid volume) was added to the system. The reaction system was mixed by gentle vortex and placed in a metal reactor, carrying out at 37 °C and under a shock condition of 900r/min for 3h. The reaction results were monitored by 15% denaturing polyacrylamide gel electrophoresis. Finally, the product was purified by the oligonucleotide extraction kit, and the purity and concentration of the product were confirmed by the NanoDrop 2000 ultramicrospectrophotometer (Thermo, USA). The final yield was 98%.

### Examples 2-12

It was done in the manner of Example 1, except that the type and/or amount of organic solvent added were varied. The results are shown in Table 2.

**Table 2**

| **No.** | **Organic solvent** | **The ratio of the volume of organic solvent to the volume of the reaction liquid (%)** | **yield (%)** |
|---|---|---|---|
| 1 | DMF | 25 | 98 |
| 2 | DMF | 5 | 58 |
| 3 | DMF | 50 | 90 |
| 4 | methyl alcohol | 5 | 50 |
| 5 | methyl alcohol | 25 | 78 |
| 6 | methyl alcohol | 50 | 65 |
| 7 | ethyl alcohol | 5 | 48 |
| 8 | ethyl alcohol | 25 | 82 |
| 9 | ethyl alcohol | 50 | 62 |
| 10 | acetone | 5 | 43 |
| 11 | acetone | 25 | 68 |
| 12 | acetone | 50 | 52 |

### Examples 13-20

It was done in the manner of Example 1, except that the type and/or amount of catalyst were varied. The experimental results are shown in Table 3.

**Table 3**

| **No.** | **copper catalyst** | **Molar ratio of catalyst to small nucleic acid sequence** | **yield (%)** |
|---|---|---|---|
| 13 | Cu(I)-TBTA | 1.5 | 62 |
| 14 | Cu(I)-TBTA | 3 | 88 |
| 15 | CuCl | 1.5 | 40 |
| 16 | CuCl | 3 | 75 |
| 17 | CuCl | 5 | 89 |
| 18 | CuBr | 1.5 | 42 |
| 19 | CuBr | 3 | 77 |
| 20 | CuBr | 5 | 86 |

### Example 21

It was done in the manner of Example 1, except that **6a** was replaced by an equimolar amount of azido-modified targeting ligand **6b.** The final yield was 96%.

### Example 22

It was done in the manner of Example 1, except that **6a** was replaced by an equimolar amount of azido-modified targeting ligand **6c.** The final yield was 95%.

### Example 23

This example is used to illustrate the efficient synthesis of KUE-PEG-siRNA conjugates targeting different genes

It was performed in the manner of Example 1, except that RNA targeting the STAT3 gene (ON**3**) was replaced by RNA targeting the PHB-1 gene (ON**5**). The final yield was 97%.

### Example 24

This example is used to illustrate the efficient synthesis of DUPA-PEG-siRNA conjugates

It was done in the manner of Example 1, except that **6a** was replaced by an equimolar amount of azide-modified DUPA ligand. The final yield was 98%.

### Example 25

The single-stranded conjugates obtained from Examples 1-22 and the Cy5-labeled complementary strand small nucleic acids were incubated at a molar ratio of 1:1 in annealing buffer (2mM magnesium acetate containing magnesium ions) at 95°C for 3 minutes to form different KUE-PEG-siRNA conjugates and DUPA-PEG-siRNA conjugates.

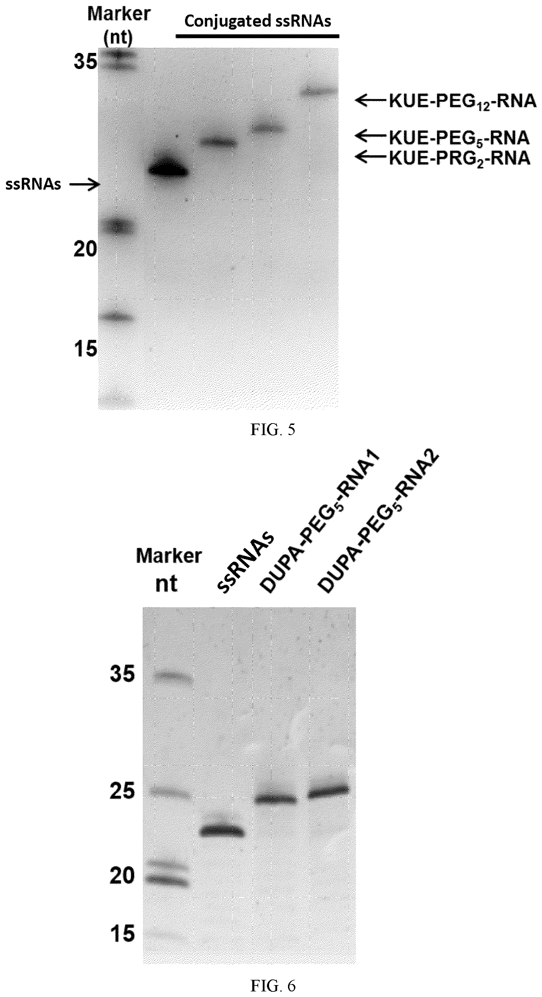

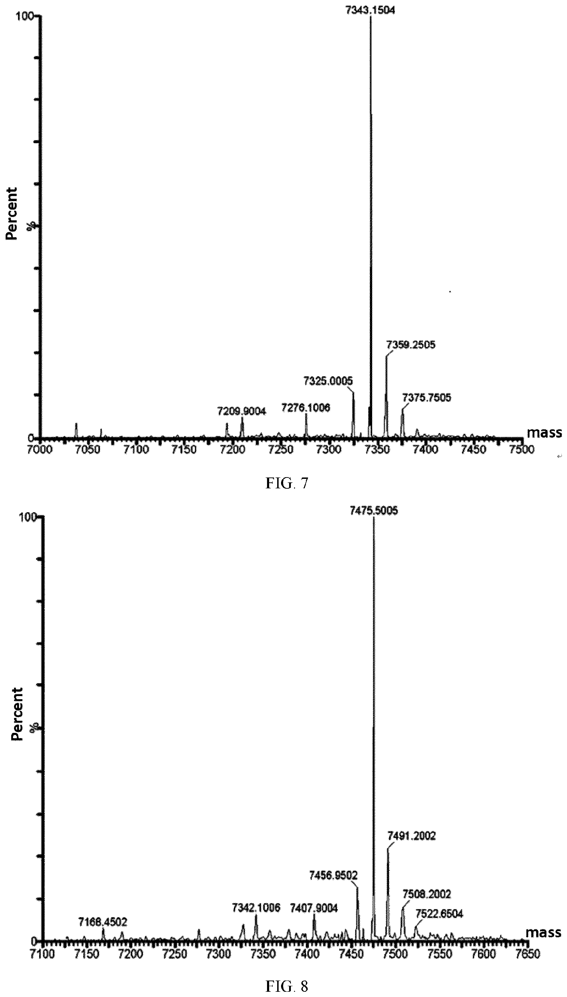

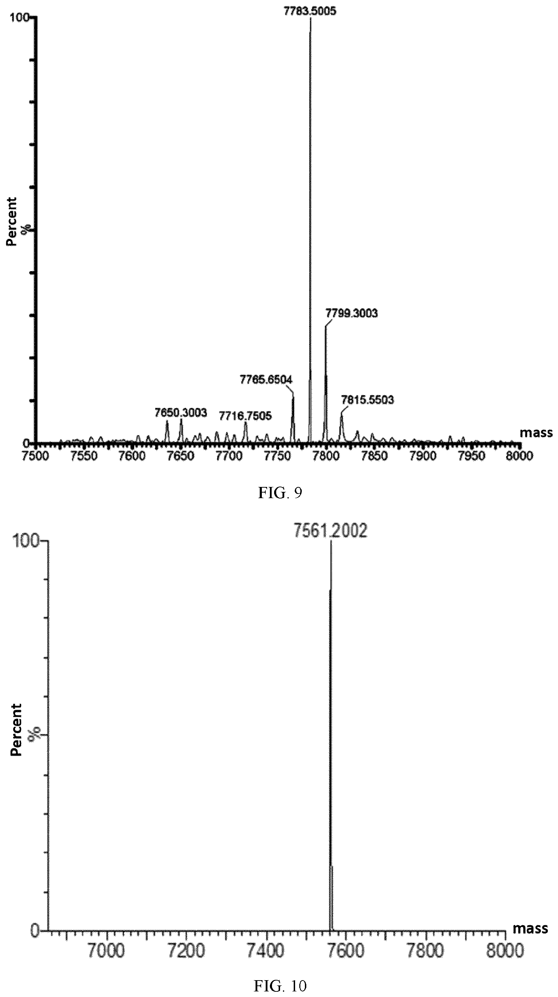

Sign diagrams of polyacrylamide gel electrophoresis results of the nucleic acid conjugate KUE-PEG₂-siRNA, nucleic acid conjugate KUE-PEG₅-siRNA, nucleic acid conjugate KUE-PEG₁₂-siRNA, and DUPA-PEG₅-siRNA obtained from Examples 1, 21, 22, 23, and 24 are shown in FIG.5 and FIG.6; Mass spectrogram are shown in Figs. 7, 8, 9, 10 (Example 23) and 11 (Example 24), respectively.

### Test Example 1

### Selectivity evaluation of KUE-PEG-siRNA conjugates for uptake in prostate cancer cells

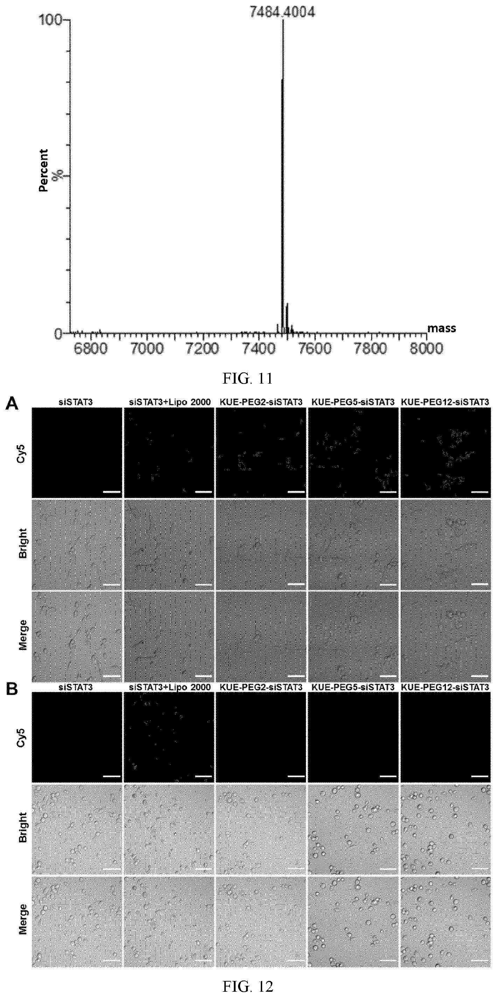

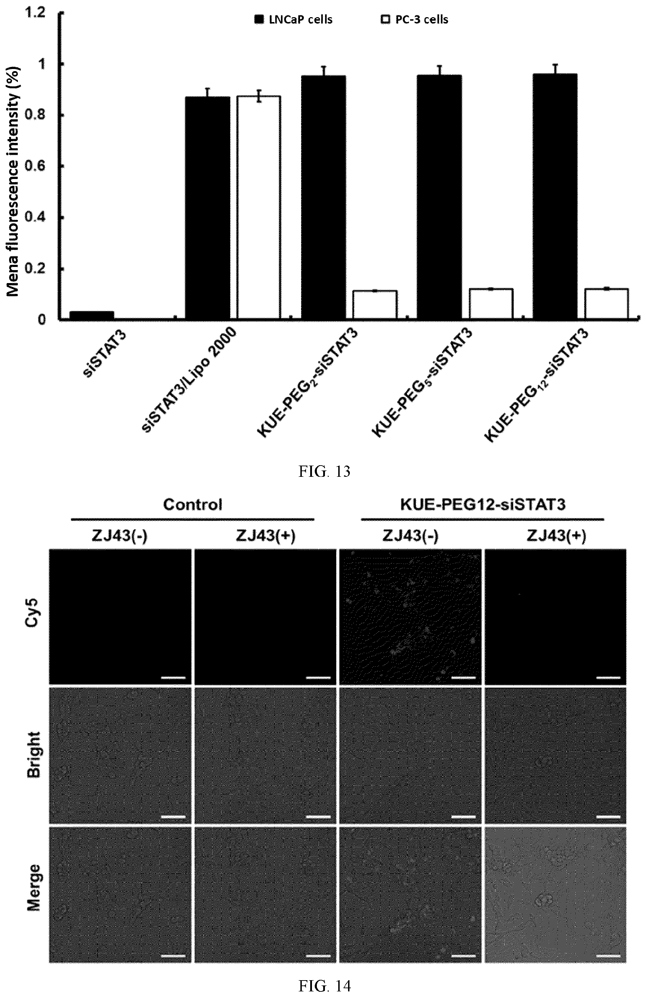

Cell imaging: LNCaP cells (PSMA-positive, purchased from Chinese Academy of Sciences) and PC3 cells (PSMA-negative, purchased from Chinese Academy of Sciences) were selected to evaluate the cellular uptake of KUE-PEG-siRNA conjugates. Cy5-labeled KUE-PEG-siRNA conjugates and siRNA (50nM) were co-incubated with both prostate cancer cells, and cells were fluorescently imaged at 649nm excitation light conditions 6h after transfection (FIG. 12A (fluorescence imaging of LNCaP cells) and FIG. 12B (fluorescence imaging of PC3 cells)). From FIG. 12A, it could be found that red fluorescent signals from the siRNA group were only observed in the presence of lipofectamine 2000 (Invitrogen, Thermo Fisher) in both types of cells, whereas red fluorescent signals from the KUE-PEG-siRNA conjugate group could be observed in LNCaP cells. In addition, in FIG. 12B, the red fluorescence signal from the KUE-PEG-siRNA conjugate group was barely observable in PC-3 cells. These results suggest that KUE-PEG-siRNA conjugates, unlike unconjugated siRNAs, are able to selectively enter prostate cancer cells and that this internalization can be accomplished with high efficiency without the help of lipofectamine 2000. The average fluorescence intensity statistics of the cell fluorescence imaging in FIG. 12A (see FIG. 13) showed that the average fluorescence intensity of the KUE-PEG-siRNA conjugate group in LNCaP cells was 95%, while that of the siRNA group transfected by lipofectamine 2000 was 82%; and the mean fluorescence intensity of KUE-PEG-siRNA conjugates in PC-3 cells was less than 5%. These results indicated that siRNAs could not enter almost any cells without the aid of transfection reagents, whereas KUE-PEG-siRNA conjugates were able to selectively enter LNCaP cells without the aid of transfection reagents.

Internalization: The pathway of KUE-PEG-siRNA conjugates into LNCaP cells was further confirmed by adding a PSMA-specific inhibitor (ZJ43, Eur. J. Neurosci., 2004, 483-494). Wherein, LNCaP cells were co-incubated with ZJ43 ahead of time, followed by addition of Cy5-labeled KUE-PEG-siRNA conjugates. At 6 h, LNCaP cells were subjected to fluorescence imaging under 640nm excitation light conditions (see FIG. 14). The results in FIG. 14 suggested that the addition of ZJ43 made the red fluorescence signal from KUE-PEG-siRNA conjugate almost undetectable in LNCaP cells, relative to the control group without PSMA specific inhibitor. This indicated that the addition of ZJ43 can significantly inhibite the uptake of KUE-PEG-siRNA conjugate by LNCaP cells, relative to the group without ZJ43 addition.

### Test Example 2

### Evaluation of uptake efficiency of KUE-PEG-siRNA conjugates in prostate cancer cells

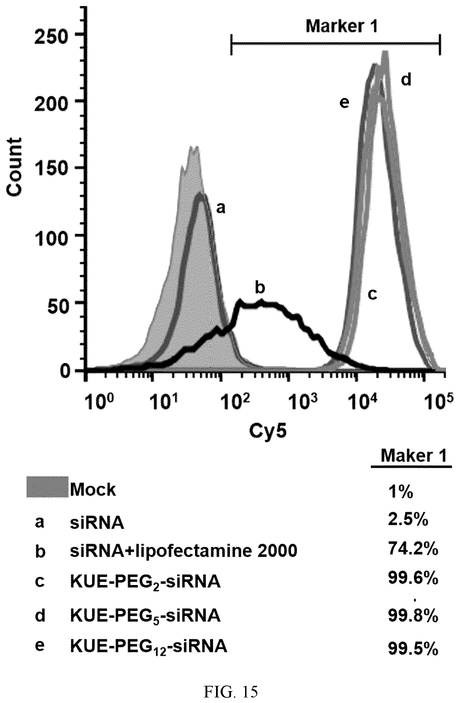

PSMA-positive expressing LNCaP cells (purchased from the Chinese Academy of Sciences) were selected to evaluate the cellular uptake efficiency of KUE-PEG-siRNA conjugates. Cy5-labeled KUE-PEG-siRNA conjugate (50nM) and siRNA (50nM) were co-incubated with LNCaP cells, wherein siRNA was co-transfected with lipofectamine 2000 (brand: Invitrogen, manufacturer: Thermo Fisher) as a control group. After 4 hours, the cells were treated according to the protocol of AnnexinV-FITC/PI apoptosis double staining kit (brand: 556547, manufacturer: BD Company, USA). The prepared cell samples were analyzed by FACS Calibur flow cytometer in the corresponding detection channel for Cy5. In the process of the experiment, the blank control group, siRNA transfection group, siRNA and lipofectamine 2000 co-transfection group, and different KUE-PEG-siRNA conjugate transfection groups were analyzed, and 10⁴ cells were collected and detected in each group. After the cell detection of all experimental groups was completed, the experimental results were analyzed using FlowJo software (FIG. 15). FIG. 15 shows that siRNA without ligand conjugation (siSTAT3) almost cannot enter LNCaP cells, which is consistent with the results of cell imaging. In addition, compared with the cell uptake efficiency of lipofectamine 2000 transfection group (74.2%), the KUE-siRNA conjugates (KUE-PEG2-siRNA, KUE-PEG5-siRNA, KUE-PEG12-siRNA) in LNCaP cells showed much higher cell uptake efficiency (>99%). These results imply that the conjugation of ligand KUE is indispensable for the delivery of siRNA into a specific prostate cancer cell line (LNCaP cells).

### Test Example 3

To verify the RNAi activity of the KUE-PEG-siRNA conjugate, a therapeutic small nucleic acid targeting the STAT3 gene was used in this test example, the sequence of which was shown in Table 4.

**Table 4**

| **ONs No.** | **sequence (SS/AS)** | |
|---|---|---|
| ON**2** | 5'- CAUGUUGUUCAGCUGCUGCUUX -3' | SEQ ID NO: 2 |

Propargyl-modified small nucleic acid sequence (ON2) (10nmol), azido-modified targeting ligand (30nmol), and Cu(I)-TBTA (50nmol) were successively added to a 200µL sterile centrifugation tube to maintain the final RNA concentration at about 100µM. DMF (25% volume of reaction liquid) was added to the system. The reaction system was mixed by gently vortex and placed in a metal reactor, carrying out at 37°C and under a shock condition of 900r/min for 3 hours. Finally, the reaction results were monitored by 15% denaturing polyacrylamide gel electrophoresis, and the single-stranded conjugates were isolated and purified by an oligonucleotide extraction kit. When the targeting ligand **6a** was used, the yield was 97.7%; when the targeting ligand **6b** was used, the yield was 96.2%; when targeting ligand **6c** was used, the yield was 95.6%.

The resulting single-stranded conjugate and complementary strand small nucleic acid were then incubated at a molar ratio of 1:1 in annealing buffer (2mM magnesium acetate) at 95 °C for 3 minutes to form KUE-PEG-siSTAT3 conjugates.

### 1) Evaluation of gene silencing effect of KUE-PEG-siSTAT3 conjugate:

Target gene silencing: KUE-PEG-siSTAT3 (KUE-PEG-siRNA) conjugates (100nM) targeting signal transduction and activator of transcription 3 (STAT3) were co-incubated with LNCaP cells.

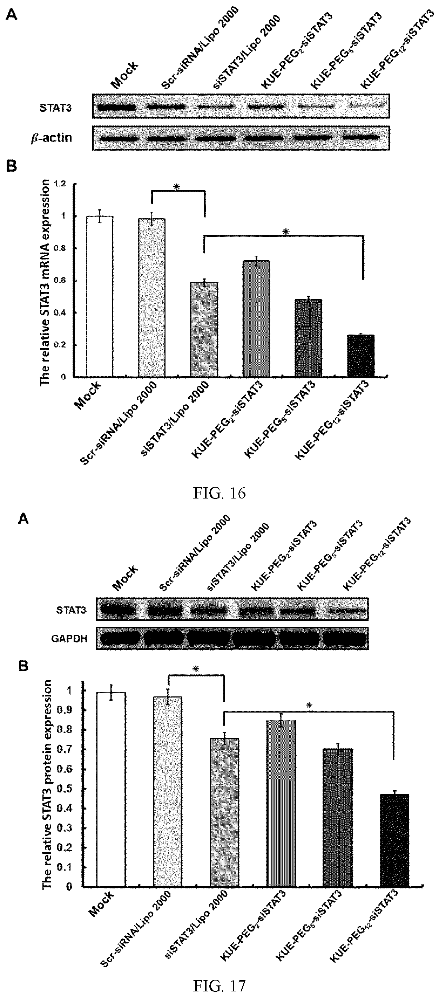

After 48 hours, mRNA expression was measured by reverse transcriptase-polymerase chain reaction (RT-PCR). As shown in FIG. 16A, all KUE-PEG-siRNA conjugates were able to effectively reduce STAT3 mRNA expression, wherein KUE-PEG5-siSTAT3 showed comparable silencing activity to siRNA transfected with the aid of lipofectamine 2000. In addition, it can be seen from FIG. 16B that the mRNA expression gradually decreased when the PEG length in the conjugate increased. These results indicated that KUE-PEG-siSTAT3 conjugate could effectively reduce the expression of the targeted gene after uptaking by LNCaP cells. Moreover, the conjugate KUE-PEG₁₂-siSTAT3 induced stronger mRNA down-regulation than KUE-PEG₂-siSTAT3 and KUE-PEG₅-siSTATS3.

Inhibition of protein expression: In addition to mRNA down-regulation experiments, the effects of the conjugates on STAT3 protein expression was explored by Western blotting (Western Blot) (see FIG. 17A and FIG. 17B). The results in FIG. 17A showed that the gray scale of the corresponding bands of all KUE-PEG-siSTAT3 conjugate experimental groups was reduced to varying degrees compared with the unconjugated siSTAT3 group, indicating that KUE-PEG-siSTAT3 conjugate can inhibit the expression of STAT3 protein. The results of quantitative experiments in FIG. 17B showed that KUE-PEG₁₂-siSTAT3 conjugate down-regulates STAT3 protein expression to the greatest extent.

### 2) Evaluation of the growth inhibition effect of KUE-PEG-siRNA conjugate in prostate cancer cells

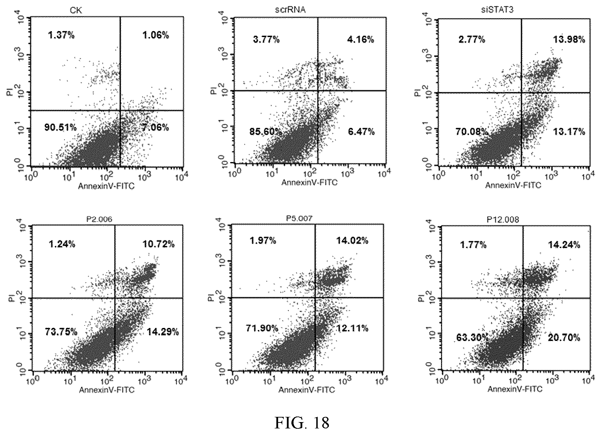

Based on the biochemical evaluation above, it was determined that KUE-PEG-siSTAT3 (KUE-PEG-siRNA) conjugate could be specifically taken up by prostate cancer cells LNCaP and induce the RNAi mechanism leading to significant reduction of STAT3 mRNA and protein expression. The effect of KUE-PEG-siSTAT3 conjugate on the growth of LNCaP cells was detected by flow cytometer (manufactor: BD, USA, model: FACS Calibur) (see FIG. 18). In FIG. 18, flow cytometry results showed that the KUE-PEG-siSTAT3 conjugates targeting STAT3 gene could promote the apoptosis of LNCaP cells to varying degrees (25.01%-34.94%), and the conjugate KUE-PEG₁₂-siSTAT3 (KUE-PEG₁₂-siRNA) could promote apoptpsis in 34.94% of LNCaP cells.

It can be seen from the above that the KUE-PEG-siSTAT3 conjugates constructed in the present disclosure can be selectively taken up by prostate cancer cells (LNCaP cells), and can trigger the RNAi mechanism in LNCaP cells to finally successfully inhibit the growth of prostate cancer cells.

Furthermore, further studies showed that the conjugates of the present disclosure were comparable to conventional means (e.g. liposomes) of delivering small nucleic acids in reducing the expression of target genes (or inhibiting the growth of cancer cells).

## Claims

1. A conjugate formed by an azido-modified targeting ligand covalently linked to a propargyl-modified siRNA sequence;
wherein the azido-modified targeting ligand has a structure as follows: 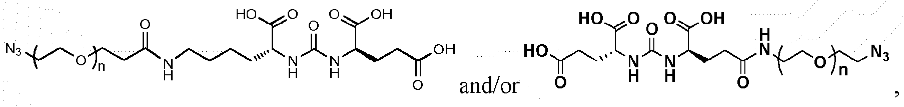 wherein n is an integer from 1 to 100;
the propargyl modification is a 3'-terminal propargyl modification;
the propargyl compound has a structure as follows: 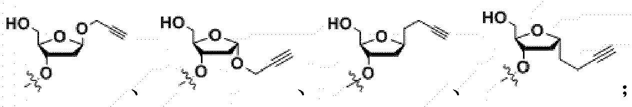
wherein, " 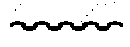 " represents the siRNA ligation site.

2. The conjugate of claim 1, wherein, the targeting ligand is able to recognize and bind to antigens expressed on the surface of target cells through glutamate; the antigen is PSMA;
and/or, the target cell is selected from at least one of prostate cancer cells, neuronal cells, renal cancer cells, and colon cancer cells.

3. The conjugate of claim 1 or 2, wherein the siRNA sequence is selected from at least one of the siRNAs targeting STAT3, PHB1, Notch1, PLK1 and BRD4;
and/or, the propargyl-modified siRNA sequence is modified to the 3'-terminal of the siRNA sequence using the phosphoramidite method by solid phase synthesis technology.

4. A method for preparating of a conjugate, comprising: making contact between an azido-modified targeting ligand and a propargyl-modified siRNA sequence in the presence of a copper monovalent catalyst;
wherein the azido-modified targeting ligand has a structure as follows: 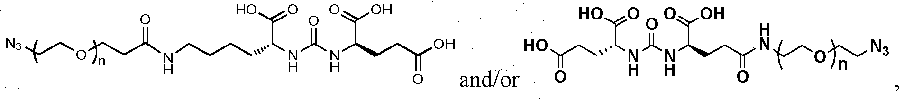 wherein n is an integer from 1 to 100;
the propargyl modification is a 3'-terminal propargyl modification;
the propargyl compound has a structure as follows: 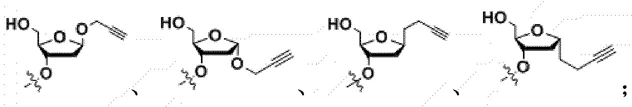
wherein, " 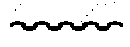 " represents the siRNA ligation site.

5. The method of claim 4, wherein the molar ratio of the azido-modified targeting ligand to the propargyl-modified siRNA sequence is (1.05-10) : 1, preferably (2-4) : 1;
and/or, the siRNA sequence is selected from at least one of the siRNAs targeting STAT3, PHB1, Notch1, PLK1 and BRD4;
and/or, the propargyl-modified siRNA sequence is modified to the 3'-terminal of the siRNA sequence using the phosphoramidite method by solid phase synthesis technology.

6. The method of claim 4 or 5, wherein the copper monovalent catalyst is selected from at least one of Cu(I)-TBTA, CuBr and CuCl, preferably Cu(I)-TBTA;
and/or, relative to 1mol of a siRNA sequence, the amount of the copper monovalent catalyst is 2-10mol, preferably 3-6mol;
and/or, the conditions for the contact comprising: temperature of 35.5-38.5 °C, preferably 36.5-37.5 °C; time of 1-5h, preferably 2-4h.

7. The method of any one of claims 4-6, wherein the contact is carried out in the presence of an organic solvent selected from at least one of N,N-dimethylformamide, methanol, ethanol, and acetone.

8. The method of any one of claims 4-7, wherein the siRNA sequence is a siRNA sequence comprising a sense strand and an antisense strand, and the method comprise:
(1) propargyl-modified sense strand and propargyl-modified antisense strand were obtained respectively;
(2) single-stranded conjugates were obtained by contacting the azide-modified target ligand with the propargyl-modified sense strand or propargyl-modified antisense strand;
(3) the conjugate was obtained by incubating the single-stranded conjugate with the other strand in annealing buffer at 90-100 °C for 1-5 minutes;
preferably, the annealing buffer is selected from a magnesium acetate solution of 1.5-2.5mM.

9. Use of the conjugate of any one of claims 1-3 or the conjugate prepared by the method of any one of claims 4-8 in the preparation of a drug for the treatment of a disease related with an abnormality in a tissue expressing PSMA, preferably occurring in glandular tissue, colon, kidney and nervous system; wherein, the glandular tissue is selected from one of the prostate, pancreas, breast and thymus.

## Patentansprüche

1. Konjugat, das durch einen Azido-modifizierten Targeting-Liganden gebildet wird, der mit einer Propargyl-modifizierten siRNA-Sequenz kovalent verknüpft ist;
wobei der Azido-modifizierte Targeting-Ligand eine Struktur wie folgt aufweist: 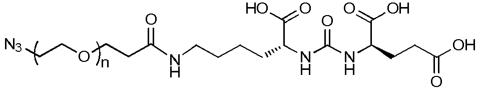 und/oder 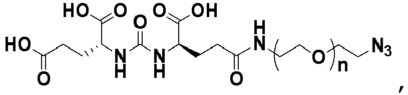 wobei n eine ganze Zahl von 1 bis 100 ist;
die Propargyl-Modifikation eine 3'-Terminus-Propargyl-Modifikation ist;
die Propargylverbindung eine Struktur wie folgt aufweist: 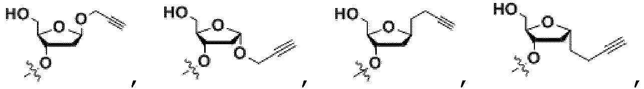
wobei " 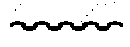 " für die siRNA-Ligationsstelle steht.

2. Konjugat nach Anspruch 1, wobei der Targeting-Ligand dazu in der Lage ist, Antigene zu erkennen und an diese zu binden, die auf der Oberfläche von Zielzellen durch Glutamat exprimiert werden; wobei das Antigen PSMA ist;
und/oder die Zielzelle aus mindestens einer von Prostatakrebszellen, neuronalen Zellen, Nierenkrebszellen und Dickdarmkrebszellen ausgewählt ist.

3. Konjugat nach Anspruch 1 oder 2, wobei die siRNA-Sequenz aus mindestens einer der siRNA ausgewählt ist, die STAT3, PHB1, Notch1, PLK1 und BRD4 targetieren;
und/oder die Propargyl-modifizierte siRNA-Sequenz unter Verwendung des Phosphoramidit-Verfahrens durch Festphasensynthese-Technologie an den 3'-Terminus der siRNA-Sequenz modifiziert ist.

4. Verfahren zur Herstellung eines Konjugats, umfassend: Herstellen eines Kontakts zwischen einem Azido-modifizierten Targeting-Liganden und einer Propargyl-modifizierten siRNA-Sequenz in Gegenwart eines einwertigen Kupfer-Katalysators;
wobei der Azido-modifizierte Targeting-Ligand eine Struktur wie folgt aufweist: 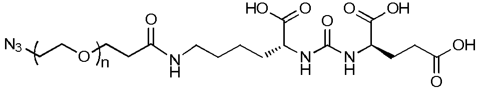 und/oder 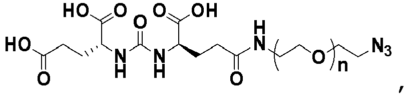 wobei n eine ganze Zahl von 1 bis 100 ist;
die Propargyl-Modifikation eine 3'-Terminus-Propargyl-Modifikation ist;
die Propargylverbindung eine Struktur wie folgt aufweist: 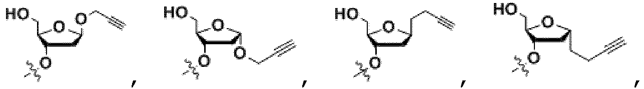
wobei " 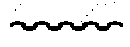 " für die siRNA-Ligationsstelle steht.

5. Verfahren nach Anspruch 4, wobei das Molverhältnis des Azido-modifizierten Targeting-Liganden zu der Propargyl-modifizierten siRNA-Sequenz (1,05-10) :1, vorzugsweise (2-4):1 beträgt;
und/oder die siRNA-Sequenz aus mindestens einer der siRNA ausgewählt ist, die STAT3, PHB1, Notch1, PLK1 und BRD4 targetieren;
und/oder die Propargyl-modifizierte siRNA-Sequenz unter Verwendung des Phosphoramidit-Verfahrens durch Festphasensynthese-Technologie an den 3'-Terminus der siRNA-Sequenz modifiziert ist.

6. Verfahren nach Anspruch 4 oder 5, wobei der einwertige Kupfer-Katalysator aus mindestens einem von Cu(I)-TBTA, CuBr und CuCl ausgewählt ist, vorzugsweise Cu(I)-TBTA ist;
und/oder bezogen auf 1 Mol einer siRNA-Sequenz die Menge des einwertigen Kupfer-Katalysators 2-10 Mol, vorzugsweise 3-6 Mol beträgt;
und/oder die Bedingungen für den Kontakt umfassen: Temperatur von 35,5-38,5°C, vorzugsweise 36,5-37,5°C; Zeit von 1-5 Std., vorzugsweise 2-4 Std.

7. Verfahren nach einem der Ansprüche 4-6, wobei der Kontakt in Gegenwart eines organischen Lösungsmittels ausgeführt wird, das aus mindestens einem von N,N-Dimethylformamid, Methanol, Ethanol und Aceton ausgewählt ist.

8. Verfahren nach einem der Ansprüche 4-7, wobei die siRNA-Sequenz eine siRNA-Sequenz ist, die einen Sense-Strang und einen Antisense-Strang umfasst, und das Verfahren umfasst:
(1) Propargyl-modifizierter Sense-Strang und Propargyl-modifizierter Antisense-Strang wurden jeweils erhalten;
(2) einsträngige Konjugate wurden durch Inkontaktbringen des Azid-modifizierten Targetliganden mit dem Propargyl-modifizierten Sense-Strang oder dem Propargyl-modifizierten Antisense-Strang erhalten;
(3) das Konjugat wurde durch Inkubieren des einsträngigen Konjugats mit dem anderen Strang in Annealing-Puffer bei 90-100°C für 1-5 Minuten erhalten;
wobei vorzugsweise der Annealing-Puffer aus einer Magnesiumacetatlösung von 1,5-2,5 mM ausgewählt wird,

9. Verwendung des Konjugats nach einem der Ansprüche 1-3 oder des Konjugats, das durch das Verfahren nach einem der Ansprüche 4-8 hergestellt wurde, bei der Herstellung eines Wirkstoffs zur Behandlung einer Krankheit, die mit einer Anomalität in einem Gewebe zusammenhängt, das PSMA exprimiert, vorzugsweise in Drüsengewebe, dem Dickdarm, den Nieren und dem Nervensystem auftretend; wobei das Drüsengewebe aus einer bzw. einem von der Prostata, der Schilddrüse, der Brust und dem Thymus ausgewählt ist.

## Revendications

1. Conjugué formé par un ligand de ciblage à modification azido lié de manière covalente à une séquence d'ARNsi à modification propargyle,
dans lequel le ligand de ciblage à modification azido possède la structure suivante : 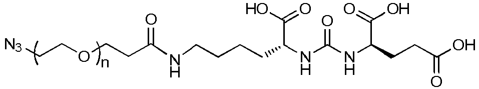 et/ou 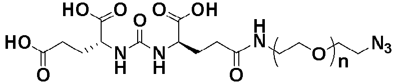
où n est un entier de 1 à 100 ;
la modification propargyle est une modification propargyle 3'-terminale ;
le composé propargyle possède la structure suivante : 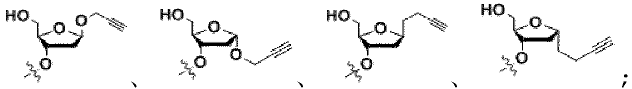
où " 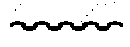 " représente le site de ligature à l'ARNsi.

2. Conjugué selon la revendication 1, dans lequel le ligand de ciblage est apte à reconnaître et à se lier à des antigènes exprimés sur la surface de cellules cibles par l'intermédiaire du glutamate ; l'antigène est le PSMA ;
et/ou la cellule cible est au moins l'une choisie parmi les cellules de cancer de la prostate, les cellules neuronales, les cellules de cancer rénal, et les cellules de cancer du côlon.

3. Conjugué selon la revendication 1 ou 2, dans lequel la séquence d'ARNsi est au moins l'une choisie parmi les ARNsi ciblant STAT3, PHB1, Notch1, PLK1 et BRD4 ;
et/ou la séquence d'ARNsi à modification propargyle est modifiée à l'extrémité 3' de la séquence d'ARNsi au moyen de la méthode au phosphoramidite par une technologie de synthèse en phase solide.

4. Procédé pour préparer un conjugué, comprenant : la réalisation d'un contact entre un ligand de ciblage à modification azido et une séquence d'ARNsi à modification propargyle en présence d'un catalyseur monovalent au cuivre ;
dans lequel le ligand de ciblage à modification azido possède la structure suivante : 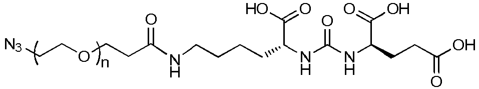 et/ou 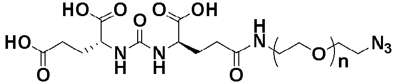
où n est un entier de 1 à 100 ;
la modification propargyle est une modification propargyle 3'-terminale ;
le composé propargyle possède la structure suivante : 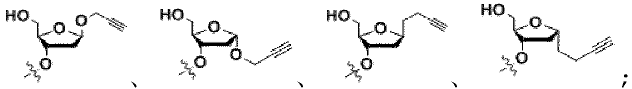
où " 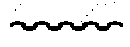 " représente le site de ligature à l'ARNsi.

5. Procédé selon la revendication 4, dans lequel le rapport molaire du ligand de ciblage à modification azido à la séquence d'ARNsi à modification propargyle est de (1,05-10)/1, de préférence de (2-4)/1 ;
et/ou la séquence d'ARNsi est au moins l'une choisie parmi les ARNsi ciblant STAT3, PHB1, Notch1, PLK1 et BRD4 ;
et/ou la séquence d'ARNsi à modification propargyle est modifiée à l'extrémité 3' de la séquence d'ARNsi au moyen de la méthode au phosphoramidite par une technologie de synthèse en phase solide.

6. Procédé selon la revendication 4 ou 5, dans lequel le catalyseur monovalent au cuivre est au moins l'un choisi parmi Cu(I)-TBTA, CuBr et CuCl, de préférence Cu(I)-TBTA ;
et/ou pour 1 mole d'une séquence d'ARNsi, la quantité du catalyseur monovalent au cuivre est de 2 à 10 moles, de préférence de 3 à 6 moles ;
et/ou les conditions pour le contact comprennent : une température de 35,5 à 38,5 °C, de préférence de 36,5 à 37,5 °C, un temps de 1 à 5 heures, de préférence de 2 à 4 heures.

7. Procédé selon l'une quelconque des revendications 4 à 6, dans lequel le contact est réalisé en présence d'un solvant organique qui est au moins l'un choisi parmi le N,N-diméthylformamide, le méthanol, l'éthanol et l'acétone.

8. Procédé selon l'une quelconque des revendications 4 à 7, dans lequel la séquence d'ARNsi est une séquence d'ARNsi comprenant un brin sens et un brin antisens, et le procédé comprend :
(1) l'obtention respectivement d'un brin sens à modification propargyle et d'un brin antisens à modification propargyle ;
(2) l'obtention de conjugués simple brin par mise en contact du ligand de ciblage à modification azido avec le brin sens à modification propargyle ou le brin antisens à modification propargyle ;
(3) l'obtention du conjugué par incubation du conjugué simple brin avec l'autre brin dans un tampon d'hybridation à 90-100 °C pendant 1 à 5 minutes ;
de préférence le tampon d'hybridation est choisi parmi une solution acétate de magnésium 1,5 à 2,5 mM.

9. Utilisation du conjugué de l'une quelconque des revendications 1 à 3 ou du conjugué préparé par le procédé de l'une quelconque des revendications 4 à 8 dans la préparation d'un médicament pour le traitement d'une maladie associée à une anomalie dans un tissu exprimant PSMA, de préférence survenant dans le tissu glandulaire, le colon, les reins et le système nerveux ; dans laquelle le tissu glandulaire est un ou plusieurs choisis parmi la prostate, le pancréas, le sein et le thymus.
